# EUROPEAN PATENT SPECIFICATION

(11) **EP 2 797 656 B1**
(45) Date of publication and mention of the grant of the patent: **29.07.2020**
(21) Application number: 12812250.4
(22) Date of filing: 20.12.2012
(51) Int. Cl.: A61M 25/00

(54) **TUBING PACKAGE FOR AN ELONGATE MEDICAL DEVICE AND METHOD MAKING THE SAME**
ROHRGEHÄUSE EINER LÄNGLICHEN MEDIZINISCHEN VORRICHTUNG SOWIE VERFAHREN ZU IHRER HERSTELLUNG
EMBALLAGE DE TUBULURE POUR UN DISPOSITIF MÉDICAL ALLONGÉ ET PROCÉDÉ DE FABRICATION ASSOCIÉ

(30) Priority: 30.12.2011 US 201161581911 P
(43) Date of publication of application: 05.11.2014
(73) Proprietor: Advant Medical Limited, Galway (IE)
(72) Inventor: MCNAMARA, Niall, Quin Co. Clare (IE)
(74) Representative: Tomkins & Co
(86) International application number: PCT/EP2012/076506
(87) International publication number: WO 2013/098215

(56) References cited:
- WO-A1-2012/141826
- WO-A2-2004/022433
- US-A- 5 840 151
- US-A1- 2006 278 546
- US-A1- 2011 127 186
- US-B1- 6 375 006
- US-B1- 6 511 573

## Description

### BACKGROUND OF THE INVENTION

### 1. Field of the Invention

The invention relates to a tubing package which can be used to house an elongate medical device such as a catheter and/or a guide wire, and/or which utilizes one or more features shown in the drawings and/or recited in the claims. A method of making and using the tubing package device is also disclosed.

### 2. Discussion of Background Information

Tubing packages for storing or packaging an elongate medical device are known. Such devices typically include tubing or lumen which is formed into a circular coiled configuration. The coiled configuration arranges or positions the tubing along a single plane via clips or tubing connectors.

Such packages are typically used to package a guide wire or catheter in a coiled configuration thereby resulting in a compact packaging system and can also safely protect the guide wire or catheter. The packaging can protect the guide wire or catheter in numerous environments such as during manufacture, assembly, packaging, shipping and use.

Such packaging devices include those disclosed in US 7,334,678 to KESLER et al. and US 2011/0127186 to ENNS et al. A tubing packaging device is also known from EP 1 551 715.

An improved device, however, would, among other things, make the tubing package more compact and improve upon the devices disclosed in KESLER, ENNS and EP '715. In the case of KESLER, an improvement would reduce or remove the spacing between the tubing coils, more securely attach the tubing connectors by over-molding the tubing connectors onto the coiled tubing, and have tubing connectors which surround the tubing coil package. In KESLER, the clips are attached from an upper or lower side and necessarily produce gaps or spacing between the coils and thus require more clips to retain a given multi-coiled configuration. Because of the spacing, the coils can move relative to each other in the bent plane and be out-of-round in areas between the tubing clips. In the case of ENNS, an improvement would reduce or remove the spacing between the tubing coils, even though ENNS teaches to attach the tubing connectors by over-molding the tubing connectors onto the coiled tubing. In ENNS, the tubing connectors surround each tubing coil, which necessarily produce gaps or spacing between the coils. Because of the spacing, the coils can move relative to each other in the bent plane and be out-of-round in areas between the tubing connectors.

US 6,511,573 to AFREMOV et al discloses a coil of tubing which can have either adjacent tubes or tubes with a space therebetween. To hold the tubes in place a material such as an adhesive can be placed across the top of the coil, on the bottom of the coil or both to hold the coil in place. The material can also envelope the coils such that the coils are banded. The coils may have a length of a straight portion of tubing prior to the coil beginning and an angled portion on the inside diameter for inserting or extracting a product in the tubing which may be similarly retained. The tubing may be coiled by insertion between two templates. The templates have injection ports for inserting material to hold or encase the tubing. Thus the tubing may be inserted in the templates, a material to hold the tubing in the coiled position inserted and the coiled tubing removed from the templates ready for use in their coiled position with the material on the tubing holding the tubing in the coiled position.

It is submitted that there is a continuing need for a tubing package of the type described herein which has one or more of the above-noted advantages and which are lacking in known devices.

### SUMMARY OF THE INVENTION

According to one aspect of the invention, there is provided a package device for an elongate medical device comprising a conduit or tube arranged in a coiled configuration, the conduit or tube having a first end and a second end ; at least one over-molded tubing connector being structured and arranged to maintain the coiled configuration; wherein the over-molded tubing connector retains more than two adjacent sections of tubing adjacent one another and without any gap or spacing therebetween; and an additional over-molded tubing connector serves as an area for locating the first conduit or tube end and the second conduit or tube end , at least one of: a portion of adjacent curved sections of the coiled configuration not covered by the at least one over-molded tubing connector contains no gap or spacing; and a portion of adjacent curved sections of the coiled configuration not covered by the at least one over-molded tubing connector contact or abut one another.

In embodiments, the elongate medical device is at least one of an elongated wire, an elongated metal rod, a solid stainless steel wire, a medical lumen, a wire insertable within a medical lumen, and a catheter.

In embodiments, the over-molded tubing connector retains three adjacent sections of tubing adjacent one another.

In embodiments, the over-molded tubing connector retains four adjacent sections of tubing adjacent one another.

In embodiments, the over-molded tubing connector retains three adjacent sections of tubing adjacent one another and without any gap or spacing therebetween.

In embodiments, the over-molded tubing connector retains four adjacent sections of tubing adjacent one another and without any gap or spacing therebetween.

In embodiments, the over-molded tubing connector comprises plural over-molded tubing connectors and one of said plural over-molded tubing connectors retains three adjacent sections of tubing adjacent one another and another of the plural over-molded tubing connectors retains four adjacent sections of tubing adjacent one another.

In embodiments, the at least one over-molded tubing connector comprises three over-molded tubing connectors each connecting three adjacent sections of tubing adjacent one another along a common plane and one over-molded tubing connector connecting four adjacent sections of tubing adjacent one another along the common plane.

In embodiments, an overall length of the conduit or tube is approximately 60 inches (152.4 cm) when straight and prior to being arranged in the coiled configuration.

In embodiments, an outer diameter of the coiled configuration is at least one of between about 3 and about 12 inches (about 7.62 and about 30.48 cm), between about 5 and about 10 inches (about 12.7 and about 25.4 cm), and between about 5 and about 8 inches (about 12.7 and about 20.32 cm).

In embodiments, coils of the coiled configuration are arranged one of on a common radial plane and each coil is spaced from a common center axis of the coiled configuration by different amounts.

In embodiments, coils of the coiled configuration are arranged one of axially one in front of another and each coil is spaced from a common center axis of the coiled configuration by substantially a same amount.

According to one non-limiting aspect of the invention, there is provided a package device for an elongate medical device, comprising a conduit or tube arranged in a coiled configuration and plural over-molded tubing connectors being structured and arranged to maintain the coiled configuration. Adjacent curved sections of the coiled configuration between the plural over-molded tubing connectors contact or abut one another.

In embodiments, the elongate medical device is at least one of an elongated wire, an elongated metal rod, a solid stainless steel wire, a medical lumen, a wire insertable within a medical lumen and a catheter.

In embodiments, at least one of the over-molded tubing connectors retains at least three adjacent sections of tubing adjacent one another and without any gap or spacing therebetween.

In embodiments, an overall length of the conduit or tube is approximately 60 inches (152.4 cm) when straight and prior to being arranged in the coiled configuration.

In embodiments, an outer diameter of the coiled configuration is at least one of between about 3 and about 12 inches (about 7.62 and about 30.48 cm), between about 5 and about 10 inches (about 12.7 and about 25.4 cm), and between about 5 and about 8 inches (about 12.7 and about 20.32 cm).

According to one non-limiting aspect of the invention, there is provided a method making the device of anyone of the types described above, wherein the method comprises arranging a conduit or tube into a coiled configuration inside or within a mold and over-molding plural tubing connectors onto the coiled configuration.

According to one non-limiting aspect of the invention, there is provided a method making a package device for an elongate medical device, wherein the method comprises arranging a conduit or tube in a coiled configuration and over-molding at least one tubing connector onto the coiled configuration. After the over-molding, adjacent curved sections of the coiled configuration contact or abut one another.

In embodiments, the over-molding comprises over-molding plural spaced apart tubing connectors onto the coiled configuration and adjacent curved sections of the coiled configuration arranged between the plural over-molded tubing connectors contact or abut one another.

According to one non-limiting aspect of the invention, there is provided a tubing package that is more compact and improves upon the devices known in the art. In particular, the device reduces or removes the spacing between the tubing coils, more securely attaches the tubing connectors by over-molding the tubing connectors onto the coiled tubing, and utilizes tubing connectors which surround the tubing coil package while allowing adjacent cured sections of the tube to abut or contact one another. Each bent section thus serves to reinforce an adjacent bent section and vice versa. Moreover, the tubing connectors are non-removable and less of them are required - unlike known packages which utilize attachable clips. Moreover, by removing the spacing, the coils generally cannot move relative to each other in the bent or common plane. As such, the problem of out-of-round areas between the tubing connectors is reduced or eliminated.

According to another non-limiting aspect of the invention, there is provided a package device for an elongate medical device, comprising a conduit or tube arranged in a coiled configuration and at least two over-molded tubing connectors being structured and arranged to maintain the coiled configuration. At least portions of adjacent curved sections of the coiled configuration between the at least two over-molded tubing connectors contain no gap or spacing.

In embodiments, the elongate medical device is at least one of; an elongated wire, an elongated metal rod, a solid stainless steel wire, a medical lumen, a wire insertable within a medical lumen, and a catheter.

In embodiments, at least one of the over-molded tubing connectors retains three adjacent sections of tubing adjacent one another.

In embodiments, at least one of the over-molded tubing connectors retains four adjacent sections of tubing adjacent one another.

In embodiments, at least one of the over-molded tubing connectors retains three adjacent sections of tubing adjacent one another and without any gap or spacing therebetween.

In embodiments, at least one of the over-molded tubing connectors retains four adjacent sections of tubing adjacent one another and without any gap or spacing therebetween.

In embodiments, one of the over-molded tubing connectors retains three adjacent sections of tubing adjacent one another and another of the over-molded tubing connectors retains four adjacent sections of tubing adjacent one another.

In embodiments, the at least two over-molded tubing connectors comprises; three over-molded tubing connectors each connecting three adjacent sections of tubing adjacent one another along a common plane, and one over-molded tubing connector retains four adjacent sections of tubing adjacent one another along the common plane.

In embodiments, an overall length of the conduit or tube is approximately 60 inches (152.4 cm) when straight and prior to being arranged in the coiled configuration.

In embodiments, an outer diameter of the coiled configuration is at least one of; between about 3 and about 12 inches (about 7.62 and about 30.48 cm), between about 5 and about 10 inches (about 12.7 and about 25.4 cm), and between about 5 and about 8 inches (about 12.7 and about 20.32 cm).

According to another non-limiting aspect of the invention, there is provided a package device for an elongate medical device, comprising a conduit or tube arranged in a coiled configuration along a common plane and plural over-molded tubing connectors being structured and arranged to maintain the coiled configuration. Adjacent curved sections of the coiled configuration between the plural over-molded tubing connectors contact or abut one another.

In embodiments, the elongate medical device is at least one of; an elongated wire, an elongated metal rod, a solid stainless steel wire, a medical lumen, a wire insertable within a medical lumen, and a catheter.

In embodiments, at least one of the over-molded tubing connectors retains three adjacent sections of tubing adjacent one another.

In embodiments, at least one of the over-molded tubing connectors retains four adjacent sections of tubing adjacent one another.

In embodiments, at least one of the over-molded tubing connectors retains three adjacent sections of tubing adjacent one another and without any gap or spacing therebetween.

In embodiments, at least one of the over-molded tubing connectors retains four adjacent sections of tubing adjacent one another and without any gap or spacing therebetween.

In embodiments, one of the over-molded tubing connectors retains three adjacent sections of tubing adjacent one another and another of the over-molded tubing connectors retains four adjacent sections of tubing adjacent one another.

In embodiments, the plural over-molded tubing connectors comprise three over-molded tubing connectors each connecting three adjacent sections of tubing adjacent one another along a common plane and one over-molded tubing connector retains four adjacent sections of tubing adjacent one another along the common plane.

In embodiments, an overall length of the conduit or tube is approximately 60 inches (152.4 cm) when straight and prior to being arranged in the coiled configuration.

In embodiments, an outer diameter of the coiled configuration is at least one of; between about 3 and about 12 inches (about 7.62 and about 30.48 cm), between about 5 and about 10 inches (about 12.7 and about 25.4 cm), and between about 5 and about 8 inches (about 12.7 and about 20.32 cm).

According to another non-limiting aspect of the invention, there is provided a package device for an elongate medical device, comprising a conduit or tube arranged in a coiled configuration along a common plane and plural over-molded tubing connectors being structured and arranged to maintain the coiled configuration. The coiled configuration comprises an inner coil, at least one intermediate coil and an outer coil. Each of the inner, intermediate and outer coils are arranged on the common plane. Adjacent curved sections of the coiled configuration between the plural over-molded tubing connectors contact or abut one another.

In embodiments, adjacent curved sections of the coiled configuration connected by the plural over-molded tubing connectors contact or abut one another.

According to another non-limiting aspect of the invention, there is provided a method of making the device of any of the types described above, wherein the method comprises arranging the conduit or tube into a coiled configuration within a mold and over-molding plural tubing connectors onto the coiled configuration.

According to another non-limiting aspect of the invention, there is provided a method of using the device of any of the types described above, wherein the method comprises inserting one end of an elongate medical device into one end of the coiled configuration.

Other exemplary embodiments and advantages of the present invention may be ascertained by reviewing the present disclosure and the accompanying drawing.

### BRIEF DESCRIPTION OF THE DRAWINGS

The present invention is further described in the detailed description which follows, in reference to the noted plurality of drawings by way of non-limiting examples of exemplary embodiments of the present invention, in which like reference numerals represent similar parts throughout the several views of the drawings, and wherein:
Fig. 1 shows a top view of a first non-limiting embodiment of a tubing package;
Fig. 2 shows a front side cross-section view of Fig. 1 along Section A-A. The tubes within the tubing connectors are not shown to illustrate the little to no wall thickness between the adjacent tube sections within the connectors;
Fig. 3 shows an enlarged part of Fig. 2 labeled Detail B;
Fig. 4 shows a left side view of Fig. 1 or a view 90 degrees from that of Fig. 2;
Fig. 5 shows the tube or conduit used in Fig. 1 in a straight orientation and in partial cross-section;
Fig. 6 shows an end view of the tube or conduit of Fig. 5;
Fig. 7 shows a top view of a second non-limiting embodiment of a tubing package;
Fig. 8 shows a top perspective view of a third non-limiting embodiment of a tubing package and illustrates tubing that is substantially transparent or translucent;
Fig. 9 shows a top view of a fourth non-limiting embodiment of a tubing package;
Fig. 10 shows a partial enlarged view of Fig. 9;
Fig. 11 shows a top perspective view of a still another non-limiting embodiment of a tubing package and illustrates tubing whose coils are oriented axially instead of on a common radial plane;
Fig. 12 shows a partial enlarged view of Fig. 11;
Fig. 13 shows a side view of Fig. 11;
Fig. 14 shows an empty mold half that can be used to over-mold connectors onto a coil of tubing in order to form a tubing package in accordance with one non-limiting embodiment of the invention;
Fig. 15 shows the mold half of Fig. 14 with one end of tubing being inserted therein;
Figs. 16 and 17 show the mold half of Fig. 14 as the tubing is almost fully inserted therein and fully inserted therein;
Figs. 18 and 19 shows the mold half of Fig. 17 with the tubing coiled therein and both before and after a second mold half is moved into contact or engagement with the first mold half;
Fig. 20 shows a cross-section view of the closed mold of Fig. 19 and illustrates how plastic resin material can be injected into the mold so as to form the over-molded connectors;
Fig. 21 shows the mold of Fig. 19 after it is opened and after the over-molded connectors have been formed and cooled sufficiently;
Fig. 22 shows the tubing package being removed from the opened mold of Fig. 20;
Figs. 23 and 24 show views of an exemplary non-limiting tubing package formed in the mold system of Figs. 14-20; and
Fig. 25 illustrates an exemplary process of forming the tubing package using the mold system of Figs. 14-20.

### DETAILED DESCRIPTION OF THE INVENTION

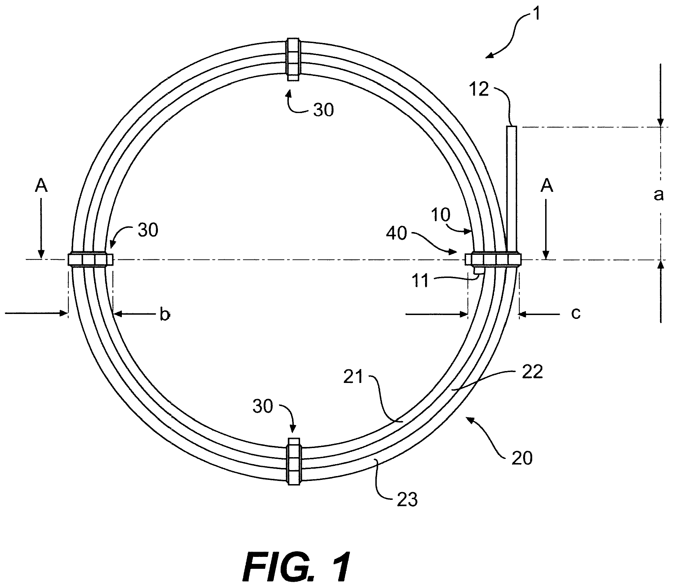

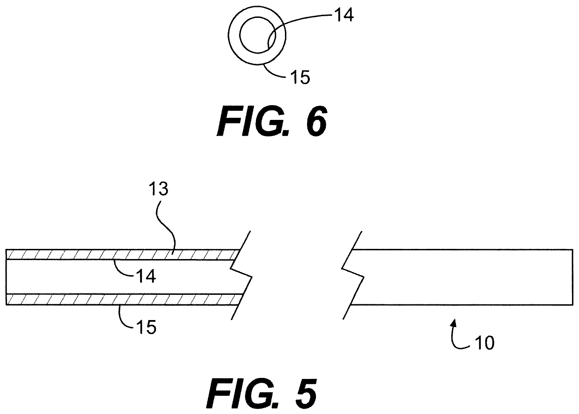

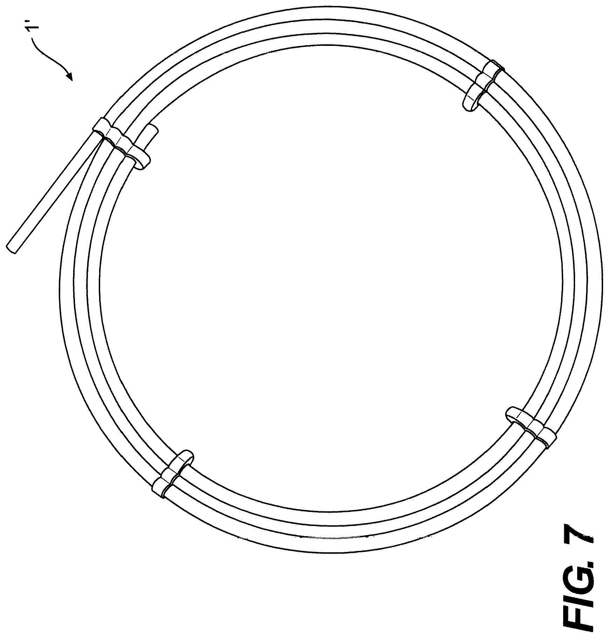

Figs. 1-7 show a first non-limiting embodiment of a tubing package or package device **1**. The package **1** is formed as a coil **20** of tubing **10** which, when straight and prior tom being coiled, can have an overall length. In one non-limiting embodiment, the overall length is about 60 inches (152.4 cm). The tubing **10** has a first end **11** which can be an open end. Alternatively, end **11** can be closed or partially closed. The tubing **10** also has a second end **12** which is an open end and which can receive therein and allow for insertion therein of an item that it to be packaged within the coil **20**. As is apparent from Figs. 6 and 7, the tubing **10** has a wall **13** which serves to protect and seal with the item or package disposed within. The wall **13** has inside generally cylindrical surface **14** as well as an outside generally cylindrical surface **15**. The inside surface **14** defines within it a space which should be sized and configured to receive therein the item and should allow for easy, unobstructed and full or complete insertion therein of the item.

As can be seen in Fig. 1, the coil of tubing **20** can include an inner winding **21** having the form of a generally curved section, e.g., circular, of tubing. The inner winding **21** is defined by the first end **11** and transitions into an intermediate winding **22** in an area where it is disposed adjacent the first end **11**. The intermediate winding **22** is defined by the previous transition area and transitions into an outer winding **23** in an area where it is disposed adjacent the first end **11**. The outer winding **23** is defined by the previous transition area and extends to the second end **12**. In embodiments, a portion of the tubing **10** extending between the second end **12** and where the tubing meets the coil section **20** is made to have a generally straight configuration for a desired length "**a**" as shown in Fig. 1. This straight section can serve to, among other things, facilitate insertion of the item or device being packaged.

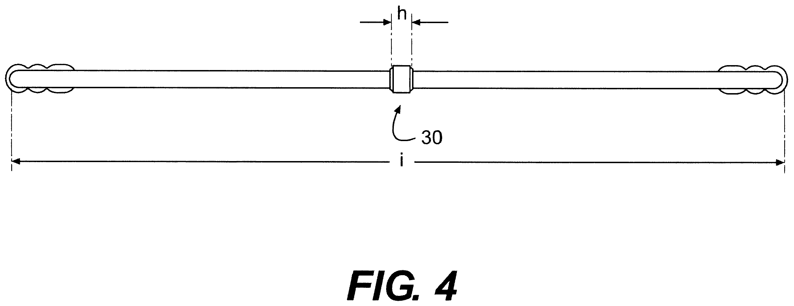

Again with reference to Fig. 1, the coil of tubing **20** can be maintained in this coiled configuration via one or more over-molded connectors **30**. In the non-limiting embodiment shown in Fig. 1, three over-molded connectors **30** are each angularly spaced by, e.g., approximately 90 degrees. An additional over-molded connector **40** is angularly spaced from one or more of the connectors **30** by, e.g., approximately 90 degrees. Also, in the non-limiting embodiment shown in Fig. 1, the additional over-molded connector **40** serves as an area for locating the tubing ends **11** and **12**. In Fig. 1, the three over-molded connectors **30** couple together three coil sections **21-23** whereas the additional over-molded connector **40** couples together the coil sections in an area of the opposite ends **11** and **12**. As can be seen in Fig. 4, an outermost diameter of the coil package 1 (measured to an outermost part of the connectors) can have value "**i**". Moreover, a width of each connector **30**/**40** can have value "**h**".

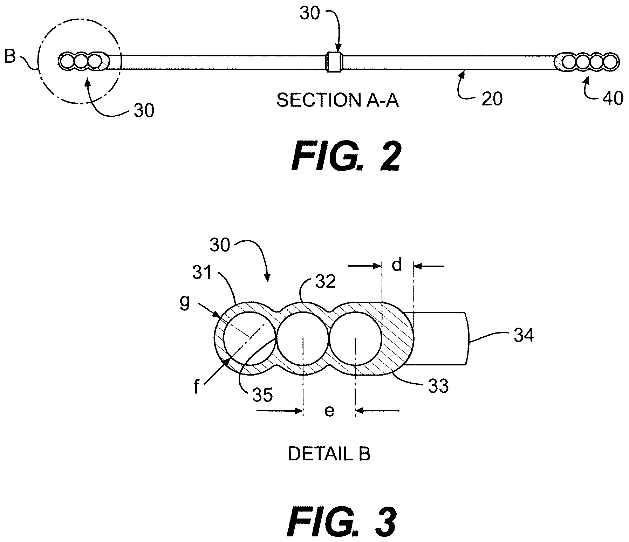

As is apparent in Fig. 1 and as is made more so in Figs. 2 and 3, the coil of tubing **20** arranges or couples together three coil sections **21-23** on a common plane such that a diameter of each coil section increases between sections **21-23**. Furthermore, the connector **30** connects together the coil sections such that substantially abut or contact one another as shown in Fig. 3. Moreover, as shown in Fig. 3, section **31** of the connector **30** surrounds most of the tubing outer diameter of part of section **21**, but does not extend into substantially the tubing outer surface contact area **35** between section **21** and **22**. Section **32** of the connector **30** surrounds only opposite sides of the tubing outer diameter of part of section **22**, but does not extend into substantially the tubing outer surface contact areas **35** between sections **21** and **22** and between sections **22** and **23**. Section **33** of the connector **30** surrounds most of the tubing outer diameter of part of section **23**, but does not extend into substantially the tubing outer surface contact area **35** between section **22** and **23**. Because of the sections **21-23** contact each other at contact area **35**, the space between these sections is essentially zero (or close thereto so that only a thin amount of connector material can extend into the area **35**) within the connectors **30** and **40**, and also in areas of the coil **20** which lack connectors **30**/**40**, i.e., the curved sections of the coil **20** between the connectors **30**/**40**. As a result of the configuration shown in Figs. 1-3, connector **30** has a width "**b**" and connector **40** has a width "**c**". Connectors **30** and **40** also have an inner surface defined by radius "**f**", an outer surface defined by radius "**g**", a width defined by dimension "**d**" and a center to center spacing defined by dimension "**e**".

According to one non-limiting example, the packaging device 1 can have the following values. Dimension "**a**" is, e.g., about 2 inches (5.08 cm). Dimension "**b**" is, e.g., about 0.6 inches (1.524 cm). Dimension "**c**" is, e.g., about 0.76 inches (1.9304 cm). Dimension "**d**" is, e.g., about 0.1 inches (0.254 cm). Dimension "**e**" is, e.g., about 0.156 inches (0.39624 cm). Dimension "**f**" is, e.g., about 0.156 inches (0.39624 cm) in diameter. Dimension "**g**" is, e.g., about 0.11 inches (0.2794 cm) in radius. Dimension "**h**" is, e.g., about 0.157 inches (0.39878 cm). Dimension "i" is, e.g., about 6.6 inches (16.764 cm).

Fig. 7 shows a second non-limiting embodiment of a tubing package **1'**. This embodiment is similar to that of Fig. 1, but shows how the tubing coil and connectors can be made to have different colors and/or shade of color and/or material. In embodiments, the material of the tubing is such that any item inserted therein need not be visible through the wall of the tubing. In embodiments, the material of the tubing is such that any item inserted therein can be visible through the wall of the tubing by making the wall substantially transparent or translucent.

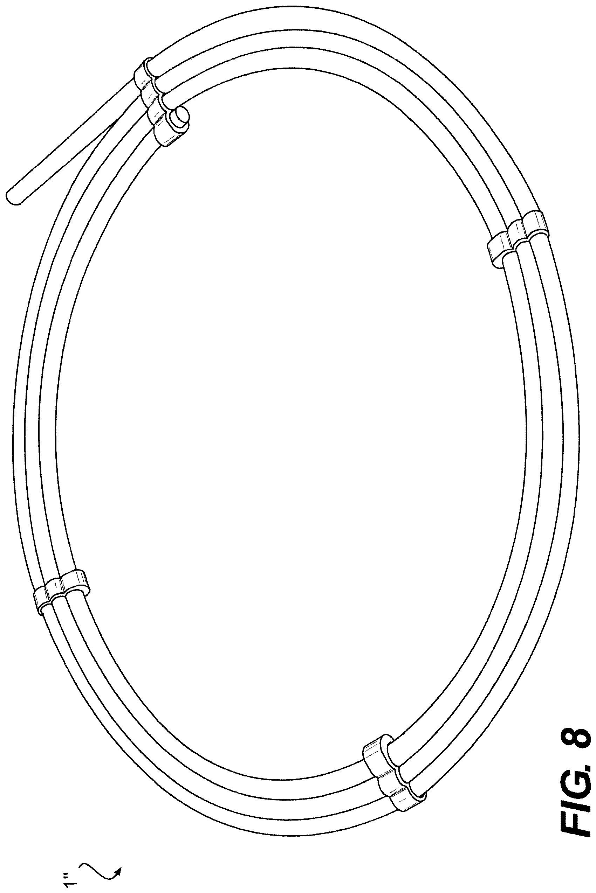

Fig. 8 shows such a non-limiting embodiment of a tubing package **1**" and illustrates tubing that is substantially transparent or translucent.

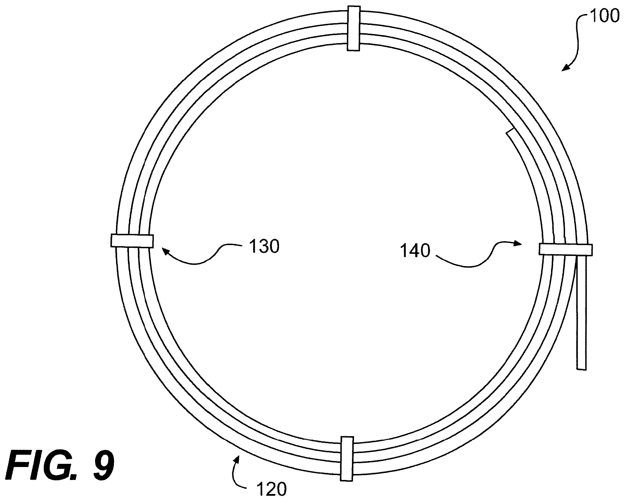

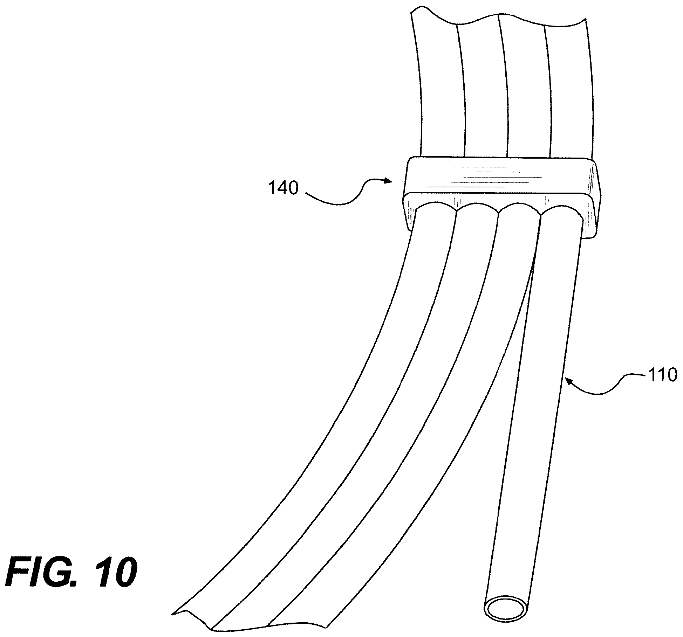

Figs. 9 and 10 show another non-limiting embodiment of a tubing package **100**. This embodiment is similar to that shown in Fig. 1 except that the first or inside end of the tubing **110** extends past the connector **140** by a greater amount and utilizes different configured connectors **130** and **140**. As can be seen in Fig. 10, the connector has a generally rectangular outer configuration. Otherwise, the package **100** utilizes a tubing coil **120** having plural substantially equally spaced over-molded connectors **130** and **140**.

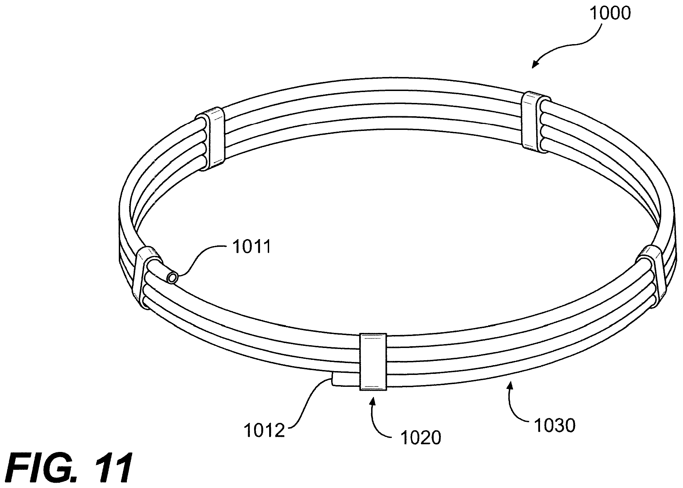

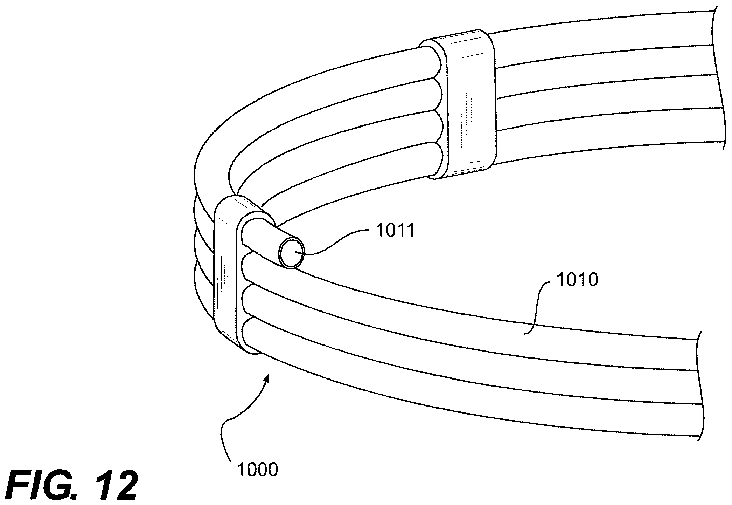

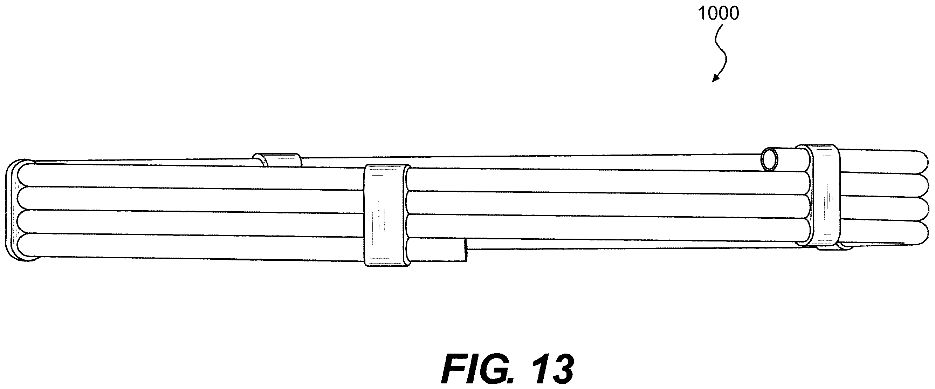

Figs. 11-13 show still another non-limiting embodiment of a tubing package or package device **1000**. The package **1000** is formed as a coil **1020** of tubing **1010** which, when straight and prior tom being coiled, can have an overall length. In one non-limiting embodiment, the overall length is about 60 inches (152.4 cm). The tubing **1010** has a first end **1011** which can be an open end. Alternatively, end **1011** can be closed or partially closed. The tubing **1010** also has a second end **1012** which is an open end and which can receive therein and allow for insertion therein of an item that it to be packaged within the coil **1020**. As is previous embodiments, the tubing **1010** has a wall which serves to protect and seal with the item or package disposed within. The wall has inside generally cylindrical surface as well as an outside generally cylindrical surface. The inside surface defines within it a space which should be sized and configured to receive therein the item and should allow for easy, unobstructed and full or complete insertion therein of the item.

In this embodiment, rather than the coils of the coiled configuration being arranged on a common radial plane or with each coil being spaced from a common center axis of the coiled configuration by different amounts, the coil sections of the coil of tubing **1020** are arranged axially one in front of another and/or each coil section is spaced from a common center axis of the coiled configuration by substantially a same amount as shown in Figs. 11-13. Furthermore, the first end **1011** is arranged in an area of one of the connectors **1030** while the second end **1012** is arranged in an area of another connector **1030** and both ends **1011** and **1012** face or are oriented toward one another. Furthermore, each connector **1030** couples together four or equal numbers of coil sections, has two oppositely arranged generally planar sides, and two oppositely arranged outwardly curved sides.

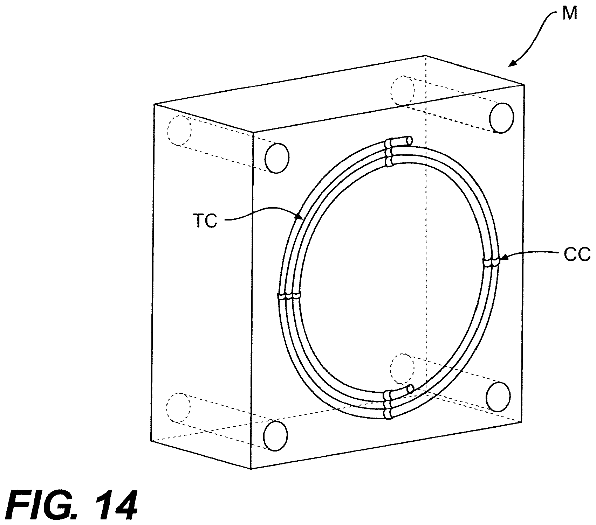

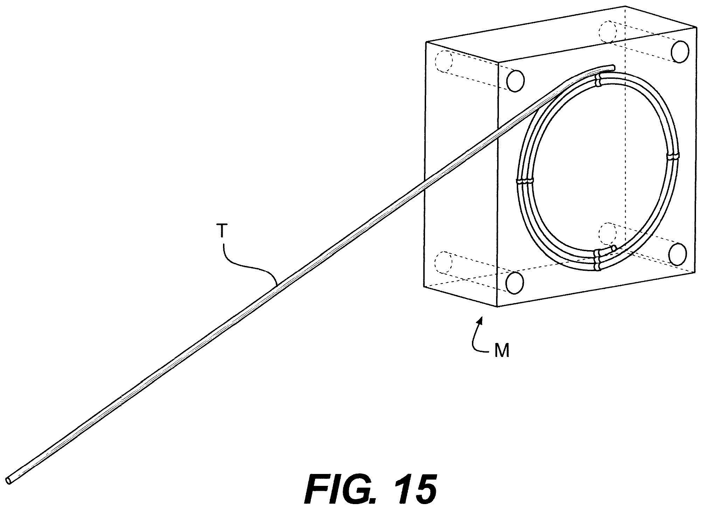

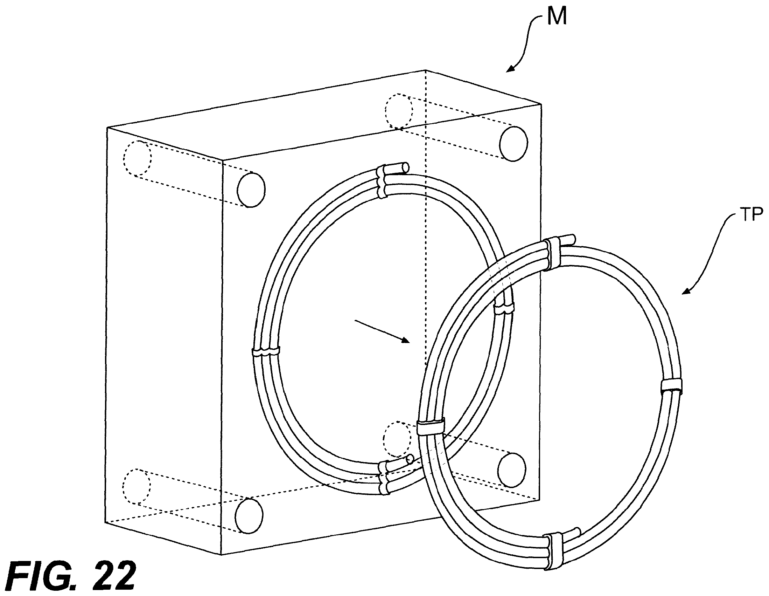

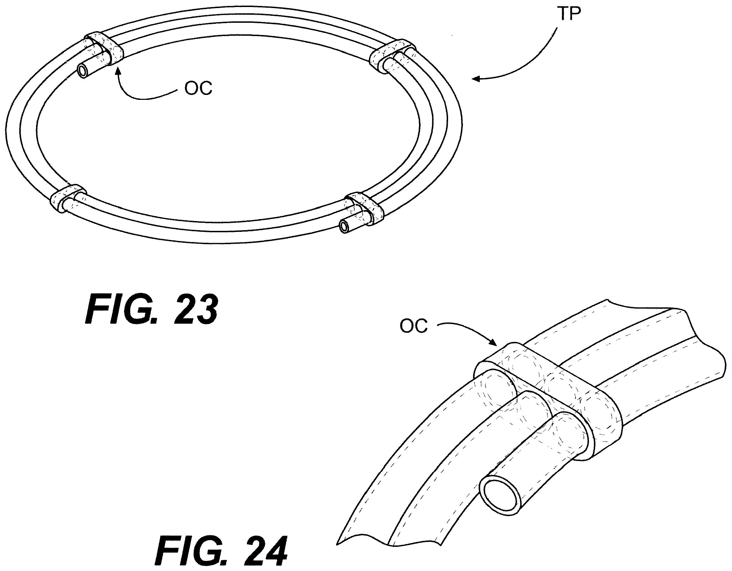

Figs. 14-22 and 25 show one non-limiting way in which the tubing package of Fig. 23 and 24 can be manufactured and/or how the coil of tubing can be over-molded with plural connectors using a mold system. This mold system and process can also be utilized to make any of the herein disclosed embodiments such as those shown in Figs. 1-13.

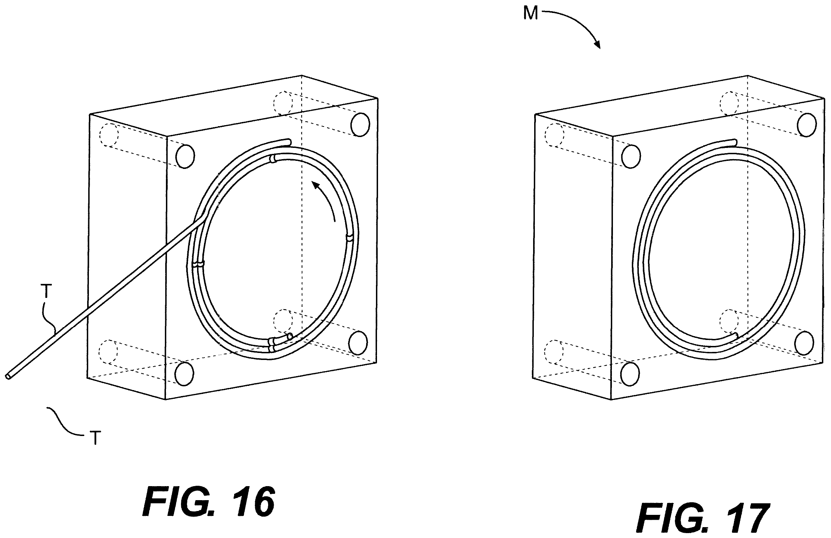

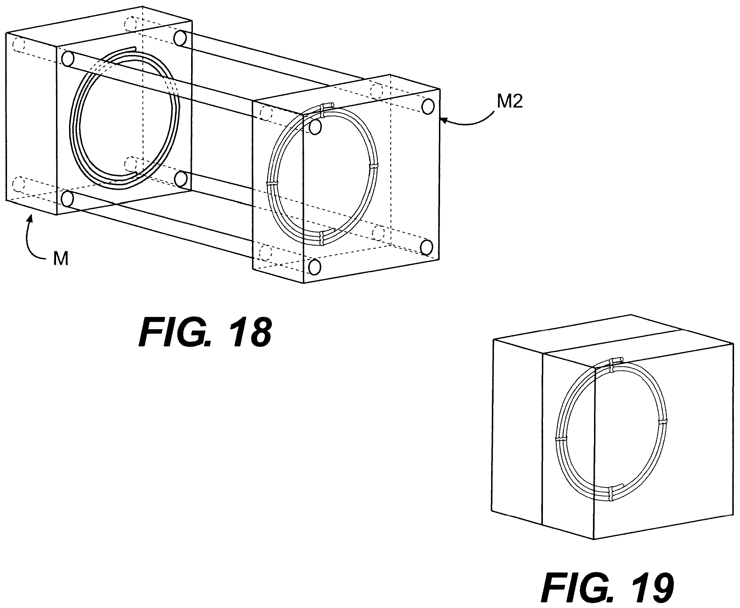

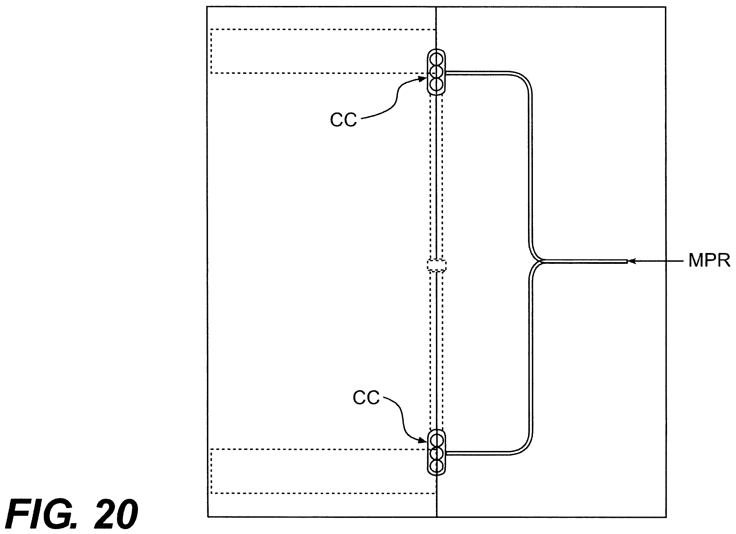

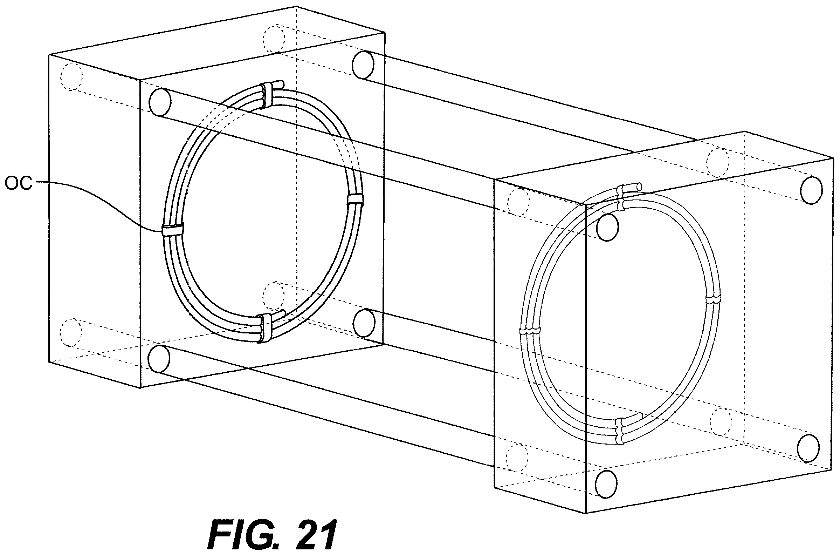

Fig. 14 shows an empty mold half **M** that can be used to over-mold connectors onto a coil of tubing in order to form a tubing package in accordance with one non-limiting embodiment of the invention. The mold **M** includes a spiral shaped tubing cavity **TC** sized and configured to receive therein the coil of tubing. Also formed in the mold **M** are over-molded connector cavities **CC** located in specific desired locations. With reference to Fig. 16, it can be seen how a specific length of tubing **T** can be initially positioned within the mold **M** and coiled into the cavity **TC**. Figs. 16 and 17 show the mold **M** as the tubing **T** is almost fully inserted therein and fully inserted therein. Next, in Figs. 18 and 19, it can be seen how a second mold half **M2**, spaced from the mold **M** with the tubing coiled therein, can be moved into contact or engagement with the mold half **M**. As can be seen in Fig. 20, once both mold half are brought into contact or closed, plastic resin material **PRM** can be injected into the mold and directed to the connector cavities **CC** so as to form the over-molded connectors **OC** shown in Fig. 21. Fig. 21 shows the mold of Figs. 19 and 20 after it is opened and after the over-molded connectors **OC** have been formed and cooled sufficiently. Next, as shown in Fig. 21, the tubing package **TP** is removed from the mold **M**.

Figs. 23 and 24 show views of an exemplary non-limiting tubing package **TP** formed in the mold system of Figs. 14-20. This embodiment is similar to the embodiments of Figs. 1-13, and locates the free opposite ends in areas of different oppositely arranged over-molded connectors **OC**.

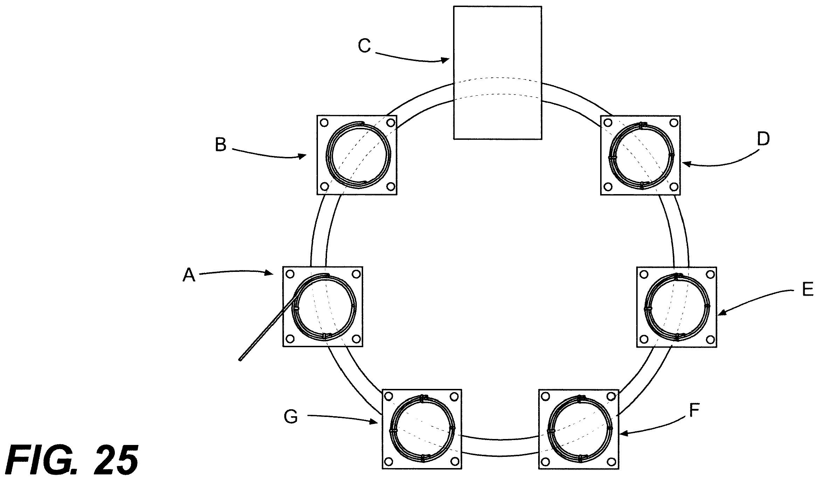

Fig. 25 illustrates an exemplary process of forming the tubing package using the mold system of Figs. 14-20. In stage **A**, the tubing is inserted or loaded into the tubing cavity of a mold. Next, in stage **B**, a checked is performed to determine if the properly loaded tubing is presented in the mold. In stage **C**, the mold is placed in an over-molding machine and plastic resin material is injected into the mold. In stage **D**, the mold is allowed to cool and opened. In stage **E**, a visual quality control inspection is performed. Next, in stage **F**, the tubing package is ejected from the mold. In final stage **G**, the empty mold is made ready to be used in stage **A**.

It is noted that the foregoing examples have been provided merely for the purpose of explanation and are in no way to be construed as limiting of the present invention. Changes may be made, within the scope of the appended claims. Although the present invention has been described herein with reference to particular means, materials and embodiments, the present invention is not intended to be limited to the particulars disclosed herein; rather, the present invention extends to all functionally equivalent structures, methods and uses, such as are within the scope of the appended claims.

## Claims

1. A package device (1) for an elongate medical device, comprising:
a conduit or tube (10) arranged in a coiled configuration, the conduit or tube (10) having a first end (11) and a second end (12);
at least one over-molded tubing connector (30) being structured and arranged to maintain the coiled configuration;
wherein the over-molded tubing connector (30) retains more than two adjacent sections of tubing adjacent one another and without any gap or spacing therebetween; and
an additional over-molded tubing connector (40) serves as an area for locating the first conduit or tube end (11) and the second conduit or tube end (12),
at least one of:
a portion of adjacent curved sections of the coiled configuration not covered by the at least one over-molded tubing connector (30/40) contains no gap or spacing; and
a portion of adjacent curved sections of the coiled configuration not covered by the at least one over-molded tubing connector (30/40) contact or abut one another.

2. The device of claim 1, wherein the elongate medical device is at least one of:
an elongated wire;
an elongated metal rod;
a solid stainless steel wire;
a medical lumen;
a wire insertable within a medical lumen;
a catheter; and
a thrombectomy device.

3. The device of claim 1, wherein said over-molded tubing connector (30/40) retains one of:
at least three adjacent sections of tubing (20) adjacent one another;
four adjacent sections of tubing (20) adjacent one another; and
five adjacent sections of tubing (20) adjacent one another.

4. The device of claim 1, wherein said over-molded tubing connector (30/40) retains three adjacent sections of tubing (20) adjacent one another and without any gap or spacing therebetween or wherein said over-molded tubing connector (30/40) retains four adjacent sections of tubing (20) adjacent one another and without any gap or spacing therebetween.

5. The device of claim 1, wherein said over-molded tubing connector (30/40) comprises plural over-molded tubing connectors (30/40) and one of said plural over-molded tubing connectors (30) retains three adjacent sections of tubing (20) adjacent one another and another of the plural over-molded tubing connectors (40) retains four adjacent sections of tubing (20) adjacent one another.

6. The device of claim 1, wherein the at least one over-molded tubing connector (30/40) comprises:
three over-molded tubing connectors (30) each connecting three adjacent sections of tubing (20) adjacent one another along a common plane; and
one over-molded tubing connector (40) connecting four adjacent sections of tubing (20) adjacent one another along the common plane.

7. The device of claim 1, wherein an overall length of the conduit or tube (20) is one of:
between 7 inches (17.78 cm) and 100 inches (254 cm); and
approximately 60 inches (152.4 cm) when straight and prior to being arranged in the coiled configuration; or
wherein an outer diameter of the coiled configuration is at least one of:
between 3 and 12 inches (7.62 and 30.48 cm);
between 5 and 10 inches (12.7 and 25.4 cm); and
between 5 and 8 inches (12.7 and 20.32 cm).

8. The device of claim 1, wherein coils of the coiled configuration are arranged one of:
on a common radial plane; and
each coil is spaced from a common center axis of the coiled configuration by different amounts.

9. The device of claim 1, wherein coils of the coiled configuration are arranged one of:
axially one in front of another; and
each coil is spaced from a common center axis of the coiled configuration by a same amount.

10. The package device for an elongate medical device of claim 1, comprising:
plural over-molded tubing connectors (30/40) being structured and arranged to maintain the coiled configuration,
wherein adjacent curved sections of the coiled configuration between the plural over-molded tubing connectors (30/40) contact or abut one another.

11. The device of claim 10, wherein the elongate medical device is at least one of:
an elongated wire;
an elongated metal rod;
a solid stainless steel wire;
a medical lumen;
a wire insertable within a medical lumen;
a catheter; and
a thrombectomy device ; or
wherein at least one of the over-molded tubing connectors (30/40) retains at least three adjacent sections of tubing adjacent one another and without any gap or spacing therebetween; or wherein an overall length of the conduit or tube is one of:
between 7 inches and 200 inches (17.78 and 508 cm); and
approximately 60 inches (152.4 cm) when straight and prior to being arranged in the coiled configuration ; or
wherein an outer diameter of the coiled configuration is at least one of:
between 3 and 12 inches (7.62 and 30.48 cm);
between 5 and 10 inches (12.7 and 25.4 cm); and
between 5 and 8 inches (12.7 and 20.32 cm).

12. A method of making the device of anyone of claims 1-11, the method comprising:
arranging a conduit or tube (20) into a coiled configuration inside or within a mold; and
over-molding plural tubing connectors (30/40) onto the coiled configuration.

13. The method making a package device for an elongate medical device of claim 12
wherein, after the over-molding, adjacent curved sections of the coiled configuration contact or abut one another.

14. The method of claim 13, wherein the over-molding comprises over-molding plural spaced apart tubing connectors (30/40) onto the coiled configuration and adjacent curved sections of the coiled configuration arranged between the plural over-molded tubing connectors (30/40) contact or abut one another.

## Patentansprüche

1. Eine Verpackungseinrichtung (1) für eine längliche medizinische Einrichtung, die Folgendes aufweist:
eine Leitung oder einen Schlauch bzw. ein Rohr (10), die oder der bzw. das in einer gewickelten Konfiguration angeordnet ist, wobei die Leitung oder der Schlauch bzw. das Rohr (10) ein erstes Ende (11) und ein zweites Ende (12) aufweist;
mindestens einen über- bzw. umspritzten Schlauchmaterialverbinder (30), der strukturiert und angeordnet ist, die gewickelte Konfiguration aufrechtzuerhalten;
wobei der umspritzte Schlauchmaterialverbinder (30) mehr als zwei benachbarte Abschnitte an Rohrmaterial aufweist, die aneinander und ohne Loch oder Abstand dazwischen aneinandergrenzen; und
wobei ein zusätzlicher umspritzter Schlauchmaterialverbinder (40) als Bereich zum Lokalisieren des ersten Leitungs- oder Schlauch- bzw. Rohrendes (11) und des zweiten Leitungs- oder Schlauch- bzw. Rohrendes (12) dient,
mindestens eines der Folgenden:
wobei ein Anteil aneinandergrenzender, gekrümmter Abschnitte der gewickelten Konfiguration, die nicht von dem mindestens einen umspritzten Schlauchmaterialverbinder (30/40) bedeckt ist, kein Loch oder keinen Abstand enthält; und
wobei ein Anteil aneinandergrenzender gekrümmter Abschnitte der gewickelten Konfiguration, die nicht von dem mindestens einen umspritzten Schlauchmaterialverbinder (30/40) bedeckt ist, einander berührt oder gegenseitig abschließt.

2. Die Einrichtung nach Anspruch 1, wobei die längliche medizinische Einrichtung mindestens eines der Folgenden ist:
ein langgezogener Draht;
ein langgezogener Metallstab;
ein Draht aus massivem, rostfreien Stahl;
ein medizinisches Lumen bzw. Lichtleiter;
ein in ein medizinisches Lumen einführbaren Draht;
ein Katheter; und
eine Thrombektomie-Einrichtung.

3. Die Einrichtung nach Anspruch 1, wobei der umspritzte Schlauchmaterialverbinder (30/40) eines der Folgenden zurückhält bzw. aufweist:
mindestens drei benachbarte Rohrmaterialabschnitte (20), die aneinander angrenzen;
vier benachbarte Rohrmaterialabschnitte (20), die aneinander angrenzen; und
fünf benachbarte Rohrmaterialabschnitte (20), die aneinander angrenzen.

4. Die Einrichtung nach Anspruch 1, wobei der umspritzte Schlauchmaterialverbinder (30/40) drei benachbarte Rohrmaterialabschnitte (20) zurückhält bzw. aufweist, und zwar aneinander angrenzend und ohne Loch oder ohne Abstand dazwischen, oder wobei der umspritzte Schlauchmaterialverbinder (30/40) vier benachbarte Rohrmaterialabschnitte (20) zurückhält bzw. aufweist, und zwar aneinander angrenzend und ohne Loch oder ohne Abstand dazwischen.

5. Die Einrichtung nach Anspruch 1, wobei der umspritzte Schlauchmaterialverbinder (30/40) eine Vielzahl umspritzter Schlauchmaterialverbinder (30/40) aufweist und wobei einer der Vielzahl von umspritzten Schlauchmaterialverbindern (30) drei benachbarte Rohrmaterialabschnitte (20) aneinander angrenzend zurückhält bzw. aufweist und wobei ein weiterer der mehreren umspritzten Schlauchmaterialverbinder (40) vier benachbarte Rohrmaterialabschnitte (20) aneinander angrenzend zurückhält bzw. aufweist.

6. Die Einrichtung nach Anspruch 1, wobei der mindestens eine umspritzte Schlauchmaterialverbinder (30/40) Folgendes aufweist:
drei umspritzte Schlauchmaterialverbinder (30), die jeweils drei benachbarte Rohrmaterialabschnitte (20) verbinden, die entlang einer gemeinsamen Ebene nebeneinander liegen; und
einen umspritzten Schlauchmaterialverbinder (40), der vier benachbarte Rohrmaterialabschnitte (20) verbindet, die nebeneinander entlang der gemeinsamen Ebene liegen.

7. Die Einrichtung nach Anspruch 1, wobei eine Gesamtlänge der Leitung oder des Rohres (20) eine der Folgenden ist:
zwischen 7 Zoll (17,78 cm) und 100 Zoll (254 cm); und
etwa 60 Zoll (152,4 cm), wenn sie gerade sind und bevor sie in die gewickelte Konfiguration gelangen; oder
wobei ein Außendurchmesser der gewickelten Konfiguration mindestens einer der Folgenden ist:
zwischen 3 und 12 Zoll (7,62 und 30,48 cm);
zwischen 5 und 10 Zoll (12,7 und 25,4 cm); und
zwischen 5 und 8 Zoll (12,7 und 20,32 cm).

8. Die Einrichtung nach Anspruch 1, wobei Wickelungen der gewickelten Konfiguration gemäß einem der Folgenden angeordnet sind:
auf einer gemeinsamen radialen Ebene; und
wobei jede Wickelung von einer gemeinsamen Mittelachse der gewickelten Konfiguration um verschiedene Beträge beabstandet ist.

9. Die Einrichtung nach Anspruch 1, wobei Wickelungen der gewickelten Konfiguration gemäß einem der Folgenden angeordnet sind:
axial voreinander; und
wobei jede Wicklung von einer gemeinsamen Mittelachse der gewickelten Konfiguration um eine gleiche Betrag beabstandet ist.

10. Die Verpackungseinrichtung für eine längliche medizinische Einrichtung nach Anspruch 1, die Folgendes aufweist:
eine Vielzahl von umspritzten Schlauchmaterialverbindern (30/40), die strukturiert und angeordnet sind, die gewickelte Konfiguration beizubehalten,
wobei Anteile aneinandergrenzender, gekrümmter Abschnitte der gewickelten Konfiguration zwischen der Vielzahl von umspritzten Schlauchmaterialverbindern (30/40) einander berühren oder gegenseitig abschließen.

11. Die Einrichtung nach Anspruch 10, wobei die längliche medizinische Einrichtung mindestens eine der Folgenden ist:
ein langgezogener Draht;
ein langgezogener Metallstab;
ein Draht aus massivem, rostfreien Stahl;
ein medizinisches Lumen bzw. Lichtleiter;
ein in ein medizinisches Lumen einführbaren Draht;
ein Katheter; und
eine Thrombektomie-Einrichtung; oder
wobei mindestens einer der umspritzten Schlauchmaterialverbinder (30/40) drei benachbarte Rohrabschnitte (20) zurückhält bzw. aufweist, und zwar aneinander angrenzend und ohne Loch oder ohne Abstand dazwischen; oder
wobei eine Gesamtlänge der Leitung oder des Rohres (20) eine der Folgenden ist:
zwischen 7 Zoll und 200 Zoll (17,78 cm und 508 cm); und
etwa 60 Zoll (152,4 cm), wenn sie gerade sind und bevor sie in die gewickelte Konfiguration gelangen; oder
wobei ein Außendurchmesser der gewickelten Konfiguration mindestens einer der Folgenden ist:
zwischen 3 und 12 Zoll (7,62 und 30,48 cm);
zwischen 5 und 10 Zoll (12,7 und 25,4 cm); und
zwischen 5 und 8 Zoll (12,7 und 20,32 cm).

12. Ein Verfahren zum Herstellen der Einrichtung nach einem der Ansprüche 1-11, wobei das Verfahren Folgendes aufweist:
Anordnen einer Leitung oder eines Schlauches bzw. eines Rohrs (20) in einer gewickelten Konfiguration in oder innerhalb einer Form bzw. Spritzgussform; und
Um- bzw. Überspritzen der Vielzahl von Schlauchmaterialverbindern (30/40) hin zur umspritzten Konfiguration.

13. Das Verfahren zum Herstellen einer Verpackungseinrichtung für eine längliche medizinische Einrichtung nach Anspruch 12, wobei, nach dem Umspritzen, aneinandergrenzende, gekrümmte Abschnitte der gewickelten Konfiguration einander berühren oder gegenseitig abschließen.

14. Das Verfahren nach Anspruch 13, wobei das Umspritzen Umspritzen einer Vielzahl von voneinander beabstandeten Schlauchmaterialverbindern (30/40) hin zur gewickelten Konfiguration aufweist und wobei aneinandergrenzende, gekrümmte Abschnitte der gewickelten Konfiguration, und zwar angeordnet zwischen der Vielzahl von umspritzten Schlauchmaterialverbindern (30/40), einander berühren oder gegenseitig abschließen.

## Revendications

1. Un dispositif d'emballage (1) pour un dispositif médical allongé, comprenant :
un conduit ou tube (10) arrangé en une configuration enroulée, le conduit ou tube (10) ayant une première extrémité (11) et une deuxième extrémité (12) ;
au moins un élément de raccordement de tube surmoulé (30) qui est structuré et agencé pour maintenir enroulée la configuration ;
dans lequel l'élément de raccordement de tube surmoulé (30) maintient plus de deux sections adjacentes de tube adjacentes entre elles et sans aucun écartement ou espacement entre elles ; et
un élément de raccordement de tube surmoulé supplémentaire (40) servant de zone pour positionner la première extrémité de tube ou de conduit (11) et la deuxième extrémité de tube ou de conduit (12),
au moins l'une parmi :
une partie des sections courbes adjacentes de la configuration enroulée non couvertes par ledit au moins un élément de raccordement de tube surmoulé (30/40) ne comprend ni écartement ni espacement ; et
une partie des sections courbes adjacentes de la configuration enroulée non couvertes par ledit au moins un élément de raccordement de tube surmoulé (30/40) est en contact ou en butée l'une avec l'autre.

2. Le dispositif selon la revendication 1, dans lequel le dispositif médical allongé est au moins l'un parmi :
un fil allongé ;
une tige métallique allongée ;
un fil d'acier inoxydable solide ;
une lumière médicale ;
un fil insérable dans une lumière médicale ;
un cathéter ; et
un dispositif de thrombectomie.

3. Le dispositif selon la revendication 1, dans lequel ledit élément de raccordement de tube surmoulé (30/40) maintient l'une de :
au moins trois sections adjacentes de tube (20) adjacentes entre elles ;
quatre sections adjacentes de tube (20) adjacentes entre elles ; et
cinq sections adjacentes de tube (20) adjacentes entre elles.

4. Le dispositif selon la revendication 1, dans lequel ledit élément de raccordement de tube surmoulé (30/40) maintient trois sections adjacentes de tube (20) adjacentes entre elles et sans aucun écartement ou espacement entre elles ou dans lequel ledit élément de raccordement de tube surmoulé (30/40) maintient quatre sections adjacentes de tube (20) adjacentes entre elles et sans aucun écartement ou espacement entre elles.

5. Le dispositif selon la revendication 1, dans lequel ledit élément de raccordement de tube surmoulé (30/40) comprend plusieurs éléments de raccordement de tube surmoulé (30/40) et l'un desdits plusieurs éléments de raccordement de tube surmoulé (30) maintient trois sections adjacentes de tube (20) adjacentes entre elles et un autre des plusieurs éléments de raccordement de tube surmoulés (40) maintient quatre sections adjacentes de tube (20) adjacentes entre elles.

6. Le dispositif selon la revendication 1, dans lequel le au moins un élément de raccordement de tube surmoulé (30/40) comprend :
trois connecteurs de tube surmoulés (30) connectant chacun trois sections adjacentes de tube (20) adjacentes entre elles dans un plan commun ; et
un élément de raccordement de tube surmoulé (40) reliant quatre sections adjacentes de tube (20) adjacentes entre elles dans le plan commun.

7. Le dispositif selon la revendication 1, dans lequel une longueur globale du conduit ou tube (20) est l'une parmi :
entre 7 pouces (17,78 cm) et 100 pouces (254 cm) ; et
approximativement 60 pouces (152,4 cm) lorsqu'il est allongé et avant d'être disposé dans la configuration enroulée ;
ou
dans lequel le diamètre extérieur de la configuration enroulée est au moins l'un :
entre 3 et 12 pouces (7,62 et 30,48 cm) ;
entre 5 et 10 pouces (12,7 et 25,4 cm) ; et
entre 5 et 8 pouces (12,7 et 20,32 cm).

8. Le dispositif selon la revendication 1, dans lequel les enroulements de la configuration enroulée sont disposés selon l'une parmi:
sur un plan radial commun ; et
chaque enroulement est espacé différemment d'un axe central commun de la configuration enroulée.

9. Le dispositif selon la revendication 1, dans lequel les enroulements de la configuration enroulée sont disposés selon l'un parmi :
axialement l'un devant l'autre ; et
chaque enroulement est espacé identiquement d'un axe central commun de la configuration enroulée.

10. Le dispositif d'emballage pour un dispositif médical allongé selon la revendication 1, comprenant :
plusieurs éléments de raccordement de tube surmoulés (30/40) qui sont structurés et agencés pour maintenir enroulée la configuration,
dans lequel des sections courbes adjacentes de la configuration enroulée entre la pluralité des éléments de raccordement de tube surmoulés (30/40) sont en contact ou en butée l'une avec l'autre.

11. Le dispositif selon la revendication 10, dans lequel le dispositif médical allongé est au moins l'un parmi :
un fil allongé ;
une tige métallique allongée ;
un fil d'acier inoxydable solide ;
une lumière médicale ;
un fil insérable dans une lumière médicale ;
un cathéter ; et
un dispositif de thrombectomie ; ou
dans lequel au moins l'un des éléments de raccordement de tube surmoulés (30/40) maintient au moins trois sections adjacentes de tube adjacentes entre elles et sans aucun écartement ou espacement entre elles ; ou dans lequel une longueur globale du conduit ou tube est l'une parmi :
entre 7 pouces et 200 pouces (17,78 et 508 cm) ; et
environ 60 pouces (152,4 cm) lorsqu'il est allongé et avant d'être disposé dans la configuration enroulée ; ou
dans lequel un diamètre extérieur de la configuration enroulée est au moins l'un parmi :
entre 3 et 12 pouces (7,62 et 30,48 cm) ;
entre 5 et 10 pouces (12,7 et 25,4 cm) ; et
entre 5 et 8 pouces (12,7 et 20,32 cm).

12. Un procédé de fabrication du dispositif selon l'une quelconque des revendications 1 à 11, le procédé comprenant :
l'agencement d'un conduit ou tube (20) dans une configuration enroulée dans ou à l'intérieur d'un moule ; et
le surmoulage de plusieurs éléments de raccordement de tube (30/40) sur la configuration enroulée.

13. Le procédé de fabrication d'un dispositif d'emballage pour un dispositif médical allongé selon la revendication 12, dans lequel, après le surmoulage, des sections courbes adjacentes de la configuration enroulée sont en contact ou en butée entre elles.

14. Le procédé selon la revendication 13, dans lequel le surmoulage comprend le surmoulage sur la configuration enroulée de plusieurs éléments de raccordement de tube surmoulés espacés (30/40) et des sections courbes adjacentes de la configuration enroulée sont disposées entre les plusieurs éléments de raccordement de tube surmoulés, (30/40) sont en contact ou en butée entre elles.
